# EUROPÄISCHE PATENTSCHRIFT

(11) **EP 0 955 067 B1**
(45) Veröffentlichungstag und Bekanntmachung des Hinweises auf die Patenterteilung: **23.06.2004**
(21) Anmeldenummer: 99890139.1
(22) Anmeldetag: 30.04.1999
(51) Int. Cl.: A61L 2/14, A61L 2/10, B23K 10/00

(54) **Verfahren und Einrichtung zum Sterilisieren von Gegenständen**
Method and apparatus for sterilizing objects
Procédé et appareil pour stériliser des objets

(30) Priorität: 04.05.1998 AT 28498 U
(43) Veröffentlichungstag der Anmeldung: 10.11.1999
(73) Patentinhaber: Inocon Technologie Gesellschaft m.b.H, 4800 Attnang-Puchheim (AT)
(72) Erfinder: Schwankhart, Gerhard, Dipl.-Ing., 4800 Attnang-Puchheim (AT)
(74) Vertreter: KLIMENT & HENHAPEL

(56) Entgegenhaltungen:
- WO-A-95/26121
- DE-A- 2 325 517
- US-A- 5 115 166
- US-A- 5 645 796

## Beschreibung

Die Erfindung bezieht sich auf ein Verfahren zum Sterilisieren von Gegenständen. Beim Sterilisieren von Gegenständen, insbesondere solchen, die auch empfindliche Dichtungen oder empfindliche Materialien enthalten, ergeben sich erhebliche Probleme. So können solche Gegenstände kaum den für eine sichere Sterilisierung Temperaturen und Einwirkzeiten, wie sie z.B. bei einer Sterilisierung mit Dampf in einem Autoklaven gegeben sind, ausgesetzt werden, ohne dass es zu einer merklichen Verkürzung der Lebensdauer des Gerätes, z.B. einer Dentalturbine kommt.

Ähnliche Probleme ergeben sich auch bei der Sterilisierung von langen Rohrleitungen, z.B. von Klimaanlagen. In solchen Fällen werden daher meist chemisch relativ aggressive Desinfektionsmittel verwendet, die oft eine relativ lange Einwirkzeit erfordern. Bei diesen ergibt sich allerdings das Problem, dass z.B. Dichtungen ebenfalls von diesen Mitteln angegriffen werden und außerdem die Gefahr besteht, dass verschiedene Keime Resistenzen gegen solche Mittel entwickeln können.

Es ist weiter bekannt, beispielsweise aus der WO 95 26121 A und aus der US 5,645,796 A, für Sterilisationsprozesse die Verwendung eines Plasmas vorzusehen. Die in diesen Druckschriften offenbarten Plasmabehandlungen der zu sterilisierenden Gegenstände erfolgen jedoch ebenfalls mit einer relativ hohen Einwirkzeit, so dass diese Verfahren nicht geeignet sind, die eingangs beschriebenen Gegenstände mit empfindlichen Dichtungen oder bestehend aus empfindlichen Materialien, zu behandeln.

Ziel der Erfindung ist es daher, diese Nacheile zu vermeiden und ein Verfahren der eingangs erwähnten Art vorzuschlagen, das eine sichere und rasche Sterilisierung bei einer möglichst weitgehenden Schonung auch empfindlicher Materialien ermöglicht. Erfindungsgemäß wird dies bei einem Verfahren der eingangs erwähnten Art durch die kennzeichnenden Merkmale des Anspruchs 1 erreicht.

Durch die vorgeschlagenen Maßnahmen wird die keimtötende Wirkung der Strahlen des Plasmas ausgenutzt, das je nach verwendetem Gas eine sehr erhebliche UV-Strahlung abgibt. Diese bewirkt eine sehr rasche Vernichtung von schädlichen Keimen. Gleichzeitig ergibt sich der Vorteil, dass nicht nur die UV-Strahlung des Plasmas für die Sterilisierung genutzt wird, sondern auch der mechanische Impuls des Plasmaimpulses und dessen thermische Wirkung. Die thermische Wirkung dieser Plasmaimpulse, deren Leistung zweckmäßigerweise 1kW bis 10kW, vorzugsweise ca. 2kW beträgt, ist aufgrund der sehr kurzen Einwirkzeit der einzelnen Impulse im wesentlichen nur auf kleinste Partikel, wie eben Bakterien und Viren gegeben, die sicher abgetötet, wogegen die Auswirkung der thermischen Belastung auf Teile im Makrobereich, wie eben kleine Dichtungen bei Dentalturbinen, trotz der hohen Temperaturen von 15 000 °C bis 50 000°C aufgrund der sehr kurzen Einwirkdauer kaum negative Auswirkungen haben.

Da in der Praxis die Plasmaimpulse mit einer sehr hohen Geschwindigkeit aus einem Plasmabrenner austreten, weisen sie auch eine sehr hohe kinetische Energie auf, wodurch sich auch der Vorteil ergibt, dass auf dem zu sterilisierenden Gegenstand keine "Bakterienleichen" verbleiben, wie dies bei einer Sterilisierung mit Elektronenoder Laserstrahlen der Fall ist.

Für die Sterilisierung von Gegenständen mit relativ engen Kanälen oder Hohlräumen ist es vorteilhaft die kennzeichnenden Merkmale des Anspruchs 2 vorzusehen. Durch diese Maßnahmen ist sichergestellt, dass sich der eingeschossene Plasmaimpuls in dem Hohlraum auch über Strecken von einigen Zentimetern fortsetzt. Für die Sterilisierung längerer enger Hohlräume ist es zweckmäßig die Merkmale des Anspruchs 3 vorzusehen. Dabei ist durch das HF-Feld sichergestellt, dass es im gesamten Hohlraum zu einer kurzzeitigen Ausbildung eines Plasmas kommt und daher eine sicherc Sterilisierung gewährleistet ist.

Durch die kennzeichnenden Merkmale des Anspruchs 4 ist es auf einfache Art und Weise möglich, den zu sterilisierenden Gegenstand mit einer Folge von sehr kurzen Plasmaimpulsen zu beaufschlagen. Dabei kommt es im Zuge der Aufladung der Kondensatorbatterie zu einem Übersteigen der Überschlagsspannung der Anoden-Kathoden-Strecke und damit zur Ausbildung eines Lichtbogens, über den es zu einer Entladung der Kondensatorbatterie kommt. Dabei verlischt der Lichtbogen, sobald die Spannung der Kondensatorbatterie unter die Brennspannung des Lichtbogens fällt. Durch entsprechende Dimensionierung der Ladeschaltung und des Entladekreises der Kondensatorbatterie im Hinblick auf die Zeitkonstanten kann die Brenndauer des Lichtbogens in jedem Zyklus, wie auch die Wiederholfrequenz festgelegt werden. Der auf diese Weise immer nur sehr kurz brennende Lichtbogen erzeugt Plasmaimpulse, die aufgrund der sehr raschen Erwärmung des umgebenden Gases mit sehr hoher Geschwindigkeit aus der Ausströmöffnung der Kammer des Plasmabrenners austreten und in den zu sterilisierenden Hohlraum eintreten bzw. in diesen eingeschossen werden. Mit der erfindungsgemäßen Einrichtung lassen sich die für dieschonende Behandlung der zu sterilisierenden Gegenstände erforderlichen kurzen Impulszeiten der Plasmaimpulse von z.B. 10⁻⁵ bis 10⁻⁴sec und eine Wiederholfrequenz von 7 bis 100Hz erreichen.

Durch die kennzeichnenden Merkmale des Anspruchs 5 kann der zu sterilisierende Gegenstand auf einfache Weise einer sehr starken UV-Strahlung ausgesetzt werden, insbesondere wenn Argon als Plasmagas verwendet wird. Dabei wird zweckmäßigerweise ein Plasmabrenner verwendet, bei dem der Lichtbogen ständig aufrecht erhalten wird, sodass das Plasma ständig vorhanden ist.

Die kennzeichnenden Merkmale des Anspruchs 6 ermöglichen lange und enge Hohlräume oder Kanäle zu sterilisieren. Dadurch ist es durch Zündung des Lichtbogens und gleichzeitige Beaufschlagung der Spule mit einem HF Signa möglich, sicherzustellen, dass sich der in den Hohlraum eingeschossene Plasmaimpuls rasch durch den gesamten Hohlraum bewegt und dadurch eine sichere Sterilisierung erreicht wird. Aufgrund der sehr kurzen Einwirkzeit des Plasmaimpulses, ist dabei eine sehr weitgehende Schonung des zu sterilisierenden Gegenstandes gewährleistet.

Um besonders kurze Plasmaimpulse erzeugen zu können, ist es vorteilhaft, die Merkmale des Anspruchs 8 vorzusehen. Diese Maßnahmen ermöglichen es, die Zündung des Lichtbogens noch vor erreichen der Überschlagsspannung der Anoden-Kathoden-Strecke auszulösen, wodurch sie die Brennzeit des Lichtbogens und damit die Brennzeit des Plasmaimpulses extrem kurz halten lässt, ohne dass ein besonders hoher Aufwand zu einer besonders niederohmigen Ausbildung des Entladekreises der Kondensatorbatterie getrieben werden muss.

Durch die Merkmale des Anspruchs 9 ist es auf einfache Art und Weise möglich auch enge und längere Hohlräume mit einem sauerstofffreien Plasmagas zu füllen, bevor ein Plasmaimpuls in diesen eingeschossen wird, wobei auf die Anordnung einer mit HF-Signalen beaufschlagbaren Spule verzichtet werden kann. Dabei können auch längere Holräume behandelt werden, da dieser eben von zwei Seiten mit Plasmaimpulsen beaufschlagt werden kann.

Für die Sterilisierung der Innenwände von Rohrleitungen, z.B. von Klimaanlagen, ist es vorteilhaft die Merkmale der Ansprüche 10 oder 11 vorzusehen.

Im ersteren Fall erfolgt die Sterilisierung durch die UV-Strahlung eines im wesentlichen gleichförmigen Plasmas und im zweiten Falle durch aus den radialen Bohrungen der Kammer des Plasmabrenners austretende Plasmaimpulse, die auf die Innenwand der zu sterilisierenden Rohrleitung auftreffen. Aufgrund der beim durch eine Aufeinanderfolge von die Überschlagsspannung der Anoden-Kathoden-Strecke des Plasmabrenners übersteigenden Spannungsimpulsen kommt es zu einer sehr hohen Ausströmgeschwindigkeit des Plasmas, sodass die einzelnen Impulse mit hoher kinetischer Energie auf die zu sterilisierende Wand der Rohrleitung auftreffen und eine rasche und sichere Sterilisierung derselben bewirken. Dabei ist durch die Pausen zwischen den einzelnen Impulsen sichergestellt, dass es auch bei empfindlichen Rohrleitungen zu keiner Beschädigung derselben kommt.

Die Erfindung wird nun anhand der Zeichnung näher erläutert. Dabei zeigen:
Fig. 1 schematisch einen Schnitt durch einen Halter mit einem eingesetzten Plasmabrenner,
Fig. 2 schematisch einen Schnitt im vergrößertem Maßstab durch den Plasmabrenner nach der Fig. 1,
Fig. 3 schematisch die elektrische Schaltung einer erfindungsgemäßen Einrichtung,
Fig. 4 und Fig. 5 schematisch einen Schnitt und eine Draufsicht auf eine erste Ausführungsform einer erfindungsgemäßen Einrichtung,
Fig. 6 eine weitere Ausführungsform einer erfindungsgemäßen Einrichtung, die zum Sterilisieren mit Impulsplasma vorgesehen ist,
Fig. 7 eine Variante der Ausführungsform nach der Fig. 6,
Fig. 8 bis 10 zwei verschiedene Ausführungsformen von erfindungsgemäßen Einrichtungen zum Sterilisieren von Rohrleitungen.

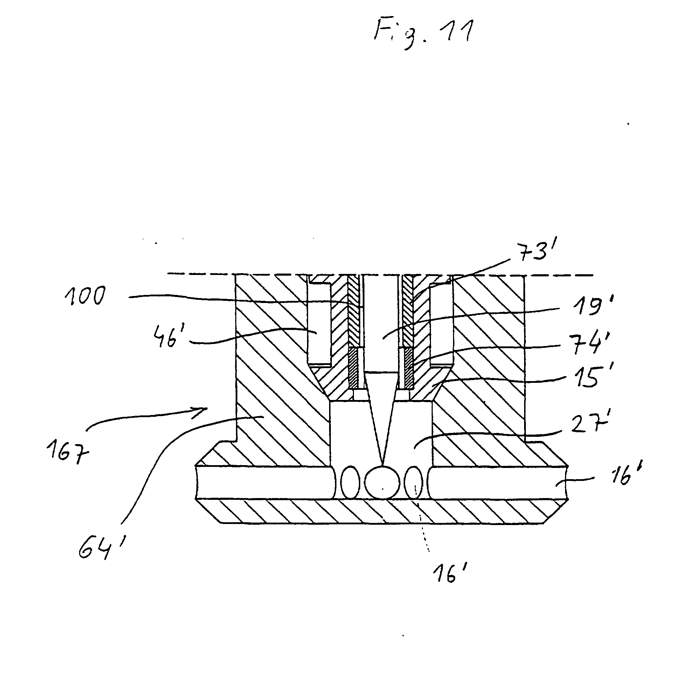

Fig. 11 einen Teilschnitt durch einen Plasmabrenner gemäß den Fig. 9 und 10 in vergrößertem Maßstab.

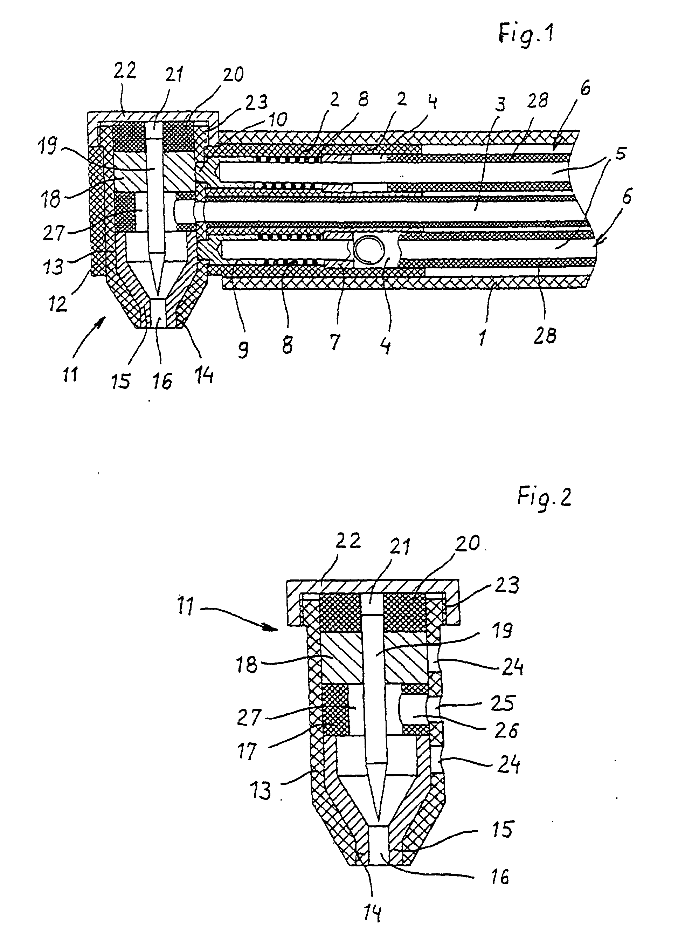

Bei der Ausführungsform nach den Fig. 1 und 2 ist ein aus einem elektrisch isolierenden Material, wie z.B. Keramik hergestellter im wesentlichen hohlzylindrischer Halter 1 vorgesehen, in dessen einem Endbereich ein ebenfalls aus einem Isoliermaterial hergestellter Einsatz 2 eingepreßt ist.

Dieser Einsatz 2 ist von einem zentralen, eine Gaszuführleitung 3 bildenden Rohr durchsetzt, das an der Stirnseite des über die Stirnseite des Halters 1 vorragenden Einsatzes 2 endet. Weiters weist der Einsatz 2 noch zwei in einer Diametralebene liegende Bohrungen 4, in denen als Widerlager dienende Einpreßteile 7 gehalten sind, die ihrerseits von den Seelen 5 von Anschlußleitungen 6 mit Spiel durchsetzt sind.

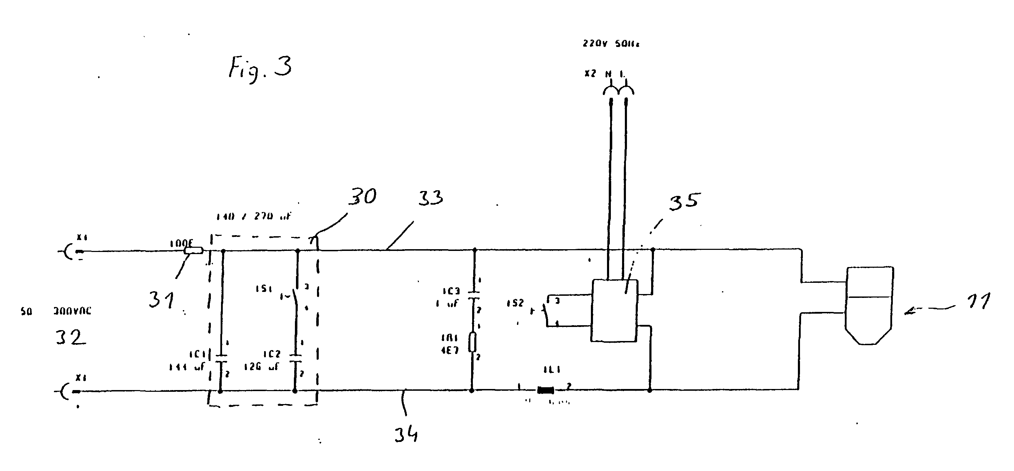

Diese Anschlußleitungen 6 sind mit einer in der Fig. 3 dargestellte Spannungsversorgung verbunden, die in einer vorgegebenen Frequenz Spannungsimpulse liefert.

An diesen Einpreßteilen 7 stützen sich Druckfedern 8 ab, die Kontaktstifte 9, die mit den Seelen 5 verlötet sind, nach außen drängen. Dabei sind die Kontaktstifte 9 an ihrem freien Ende mit einem stirnseitigen Ansatz 10 versehen, der mit einer Kontaktfläche eines Plasmabrenners 11 zusammenwirkt, der in einer an der Stirnseite des Halters 1 angeordneten Befestigungseinrichtung 23 gehalten ist, die als ein aus einem elektrisch isolierenden Material hergestellten Bügel ausgebildet ist, in den der Plasmabrenner 11 von oben her eingesetzt ist.

Dieser Plasmabrenner 11 weist einen Verbindungsteil 13 aus einem elektrisch isolierenden Material, z.B. Keramik auf, der in seinem unteren Bereich kegelförmig verjüngend ausgebildet ist und an seiner unteren Stirnseite eine Öffnung 14 aufweist.

Diese Öffnung 14 ist von einer ringförmigen Anode 15 durchsetzt, die in üblicher Weise aus einem elektrisch leitenden und thermisch hoch belastbaren Material hergestellt ist und in ihrem Mündungsbereich eine Düsenöffnung 16 aufweist.

Die Anode 15 weist einen sich nach oben zu konisch erweiternden Bereich auf, der innen an dem Verbindungsteil 13 anliegt und der in einen zylindrischen Bereich übergeht.

An der oberen Stirnseite der Anode 15 liegt ein Zwischenteil 17 an, der ringförmig ausgebildet und aus einem elektrisch isolierenden Material, z.B. Keramik, hergestellt ist.

An der oberen Stirnseite des Zwischenteiles 17 liegt ein aus einem elektrisch gut leitenden Material, z.B. Kupfer, hergestellter Halteteil 18 an, in dem eine Kathode 19 eingepreßt ist, die aus einem elektrisch leitenden und thermisch hoch belastbaren Material, wie z.B. Wolfram-Ceroxid-Legierung hergestellt ist und in ihrem der Düsenöffnung 16 der Anode 15 nahen Endbereich konisch ausgebildet ist.

Die Anode 15, wie auch der Halteteil 18 sind zur Festlegung der gegenseitigen Lage der Kathode 10 und der Düsenöffnung 16 der Anode zweckmäßigerweise in den Verbindungsteil 13 eingepaßt.

Die Anode 15, der Zwischenteil 17 und der Halteteil 18 mit der eingepreßten Kathode 19 bilden dabei gemeinsam mit dem Verbindungsteil 13 einen Modul des Gerätes, der leicht in den Halter eingebaut und aus diesem wieder entfernt werden kann.

Auf der oberen Stirnseite es Halteteiles 18 liegt ein aus einem Isoliermaterial hergestellter Druckteil 20 an, der eine die Kathode 19 mit Spiel aufnehmende Bohrung 21 aufweist und über die Stirnseite des Verbindungsteiles 13 vorragt.

Dieser Druckteil 20 wirkt mit einem Deckel 22 zusammen der auf ein im der oberen Stirnseite des Verbindungsteiles 13 nahen Bereich angeordneten Außengewindes 23 aufgeschraubt ist.

Der Verbindungsteil 13 ist mit drei entlang einer Mantellinie angeordneten radialen Bohrungen 24, 25 versehen, von denen die Bohrungen 24 den Durchtritt der Ansätze 10 der Kontaktstifte 9 ermöglichen und im Bereich des Halteteiles 18, bzw. der Anode 15 liegen. Die Bohrung 25 ist im Bereich des Zwischenteiles 17 angeordnet und fluchtet mit einem radial verlaufenden Einlaß 26 des Zwischenteiles der zu einer durch die Innenwand des Zwischenteiles 17 begrenzten Kammer 27 führt, die von der Kathode 19 durchsetzt ist.

Dabei fluchtet die Bohrung 25 bei in den Halter 1 eingesetztem Plasmabrenner, der als Modul aufgebaut ist, auch mit der im Halter 1 vorgesehenen Gaszuführleitung 3.

Zum Einbau des als Modul aufgebauten Plasmabrenners 11 genügt es die Anschlußleitungen 6, deren Isoliermäntel 28 mit Spiel in den Bohrungen 4 des Einsatzes 2 des Halters 1 geführt sind, zurückzuziehen und den Plasmabrenner 11 von oben in den Bügel 12 einzusetzen. Danach können die Anschlußleitungen 6 losgelassen werden und die Kontaktstifte 9 rasten in die Bohrungen 24 des Verbindungsteiles 13 ein und sichern die Lage des Plasmabrenners 11 im Halter 1. Gleichzeitig werden sich mit ihren Stirnflächen mittels der Federn 8 an den Halteteil 8, bzw. die Anode 15 angepreßt und so ein guter elektrischer Kontakt hergestellt.

Beim Betrieb des Plasmabrenners 11 kann über die Gaszuführleitung 3 ein Gas, z.B. Helium, Stickstoff u.a., in die Kammer 27, die u.a. auch von der die Düsenöffnung 16 bestimmenden Anode 15 begrenzt ist, eingeleitet, das die Kathode 19 umspült und diese im Betrieb gleichzeitig kühlt.

Wird nun ein Spannungsimpuls, dessen Spannung über der Überschlagsspannung der Strecke Anode 15 - Kathode 19 liegt, so bildet sich ein Lichtbogen aus, der ein Plasma erzeugt, das aus der Düsenöffnung 16 austritt. Sinkt die an der Kathode 19 und der Anode 15 anliegende Spannung unter die Brennspannung des Lichtbogens ab, so erlischt dieser und der Stromfluß über die Anoden-Kathoden-Strecke wird unterbrochen.

Grundsätzlich ist jedoch zu bemerken, daß eine Einleitung von Gas in die Kammer 27 nicht unbedingt erforderlich ist und diese auch keine Bohrung 25 aufweisen muß. In einem solchen Fall saugt die Kammer 27 nach dem Ausstoßen eines Plasmaimpulses nach dem Verlöschen des Lichtbogens aus der Umgebung Luft an. Beim nachfolgenden Zünden eines neuen Lichtbogens aufgrund des Anlegens eines weiteren Spannungsimpulses wird die Luft durch den Lichtbogen ionisiert und rasch erwärmt. Wodurch sie sich entsprechend rasch ausdehnt und mit hoher Geschwindigkeit aus der Düsenöffnung 16 ausströmt.

Eine Spannungsversorgung für einen Plasmabrenner nach den Fig. 1 und 2 ist in der Fig. 3 dargestellt, wobei die Spannungsversorgung zur Erzeugung eines Impulsplasmas vorgesehen ist.

Dabei ist eine Kondensatorbatterie 30 über einen Ladewiderstand 31 mit den Anschlüssen X1 einer regelbaren Gleichspannungsquelle 32 verbunden. Die Kondensatorbatterie 30 weist einen fest angeschlossenen Kondensator 1C1 und einen über einen Schalter 1S1 zu diesem parallel zuschaltbaren Kondensator 1C2 auf, wobei es sich in beiden Fällen auch um Gruppen von Kondensatoren handeln kann.

Diese Kondensatorbatterie 30 ist über Anschlußleitungen 33, 34 mit dem Plasmabrenner 11, bzw. dessen in der Fig. 3 nicht dargestellten Kathode und Anode verbunden.

Parallel zur Kondensatorbatterie 30 ist ein R/C-Glied geschaltet, das durch einen Kondensator 1C3 und einen Widerstand 1R1 gebildet ist. Dieses R/C-Glied bildet in Verbindung mit der in der Anschlußleitung 34 geschalteten Drossel 1L1 einen HF-Sperrkreis, der zum Schutz der Kondensatorbatterie 30 vor HF-Signalen vorgesehen ist.

Weiters sind noch die Ausgänge eines Zündgerätes 35 an die Anschlußeitungen 33, 34 angeschlossen. Dieses Zündgerät 35 ist eingangsseitig mit einer Wechselspannungsquelle X2 verbunden und mit einem Triggerschalter 1S2 versehen, durch dessen Betätigung ein Zündimpuls auslösbar ist.

Beim Betrieb kommt es zur Aufladung der Kondensatorbatterie 30 entsprechend der eingestellten Spannung der Gleichspannungsquelle 32, die z.B. zwischen 50V und 300V einstellbar ist, und der durch die Kapazität der Kondensatorbatterie 30 und die Leitungswiderstände und den Ladewiderstand mitbestimmten Zeitkonstante.

Erreicht die Kondensatorbatterie eine Spannung, die der Überschlagsspannung der Anoden-Kathoden-Strecke 15, 19 des Plasmabrenners 11 entspricht, so kommt es zum Zünden eines Lichtbogens zwischen Anode 15 und Kathode 19 (Fig. 2) und damit zur Bildung von Plasma in der Kammer 27 des Plasmabrenner 11.

Gleichzeitig entlädt sich die Kondensatorbatterie 30 entsprechend der durch deren Kapazität und den Leitungswiderständen und dem Widerstand des Lichtbogens gegebenen Zeitkonstante. Sinkt durch diese Entladung die Spannung der Kondensatorbatterie 30 unter die Brennspannung des Lichtbogens ab, so erlischt dieser und die Kondensatorbatterie 30 lädt sich wieder auf, wodurch sich der beschriebene Vorgang wiederholt und sich eine Frequenz ergibt, die durch Lade- und Entlade-Zeitkonstanten bestimmt ist. Dabei ist der Betrieb des Zündgerätes nicht erforderlich.

Für bestimmte Anwendungen kann es erwünscht sein. Den Zündzeitpunkt des Lichtbogens genau zu bestimmen oder einen solchen vor Erreichung der Überschlagsspannung der Anoden-Kathoden-Strecke auszulösen, um besonders kurze Plasmaimpulse erzeugen zu können.

In diesem Fall wird durch Betätigung des Triggerschalters 1S2 ein Zündimpuls ausgelöst, der zur Zündung eines Lichtbogens zwischen der Anode 15 und der Kathode 19 des Plasmabrenners 11 führt, ohne daß die Kondensatorbatterie 30 eine der Überschlagsspannung dieser Strecke entsprechende Spannung erreicht hat. Auf diese Weise kann auch das Tastverhältnis, das z.B. zwischen 1:10 und 1:100 und darüber hinaus gewählt werden kann, entsprechend verändert werden und das Verhältnis zwischen der Brenndauer des Lichtbogens und dessen Brennpause während eines Zyklusses im Sinne einer Verlängerung der Brennpause verändert werden, da die Energie der hochfrequenten Zündimpulse des Zündgerätes zwar zum Zünden des Lichtbogens, nicht aber zu dessen Aufrechterhaltung ausreicht, wenn die Spannung der Kondensatorbatterie 30 unter der Brennspannung des Lichtbogens abgesunken ist.

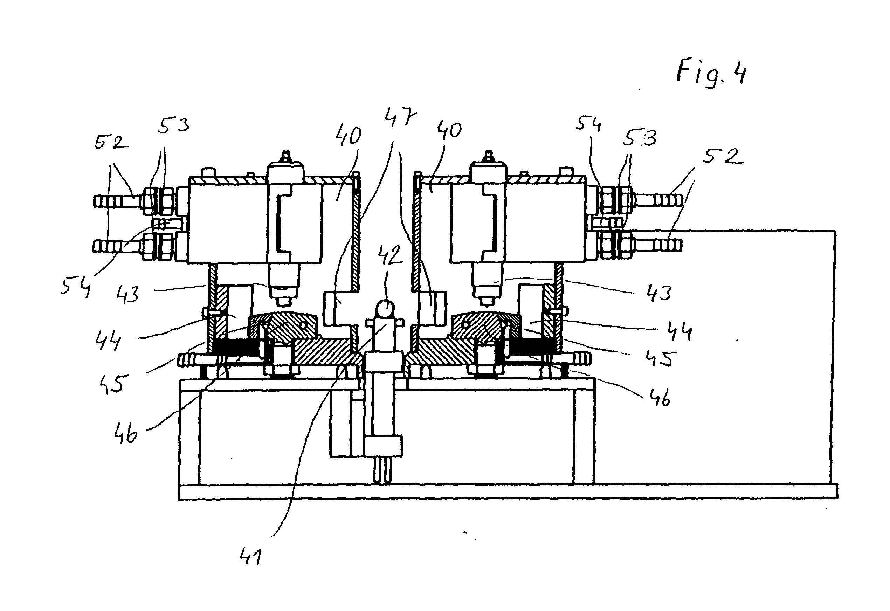

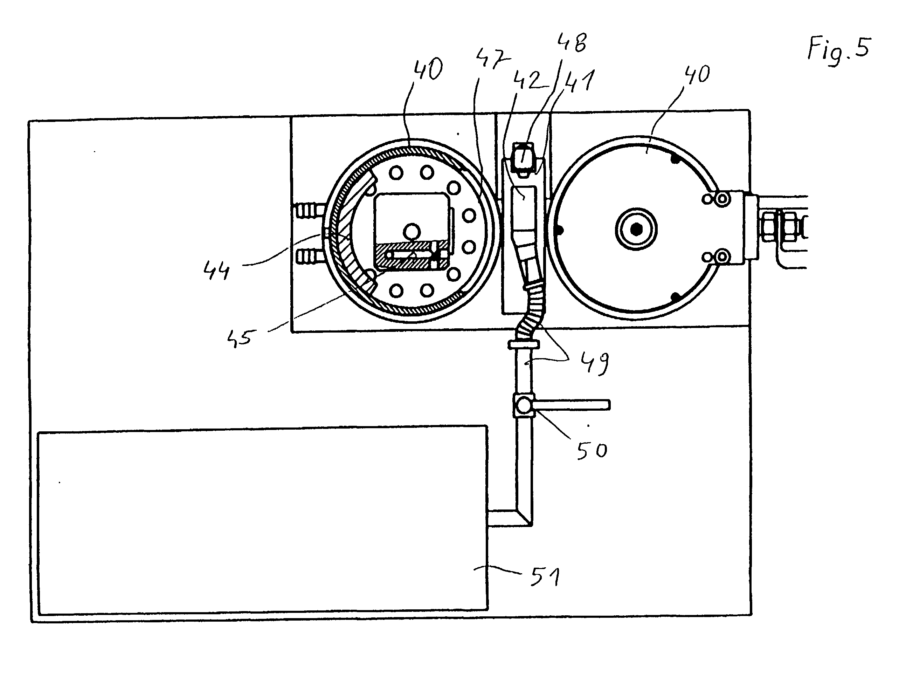

Die Fig. 4 und 5 zeigen eine erste Ausführungsform einer erfindungsgemäßen Einrichtung zu Sterilisieren, wobei ein ein Flow-Plasma, bzw. ein im wesentlichen kontinuierliches Plasma liefernder Plasmabrenner vorgesehen ist.

Bei dieser Einrichtung sind zwei Gehäuse 40 vorgesehen, die zu beiden Seiten einer Aufnahme 41 angeordnet sind, die zur Aufnahme eines zu sterilisierenden Werkstückes 42 dient.

In jedem Gehäuse 40 ist ein Plasmabrenner 43 gehalten, der in seinem wesentlichen Aufbau dem in den Fig. 1 und 2 dargestellten entsprechen kann. Als Spannungsversorgung ist dabei eine übliche, zweckmäßigerweise regelbare, Spannungsquelle, die eine im wesentlichen konstante, die Brennspannung des Lichtbogens zwischen der Anode 15 und der Kathode 19 übersteigende Spannung liefert, in Verbindung mit einer Zündeinrichtung vorgesehen. Dadurch kann bei zugeschalteter Spannungsversorgung mittels der Zündeinrichtung ein Lichtbogen gezündet und Plasma erzeugt werden, wobei dann der Lichtbogen auch nach dem Abklingen des Zündimpulses, bzw. der Zündspannung aufgrund der Versorgung mit einer die Brennspannung übersteigenden Spannung brennen bleibt.

Die Plasmabrenner 43 sind über die Anschlüsse 52 mit einem nicht dargestellten Kühlmittelkreislauf verbunden, wobei auf diesen auch die elektrischen Anschlüsse 53 zur Verbindung mit der Spannungsquelle angeordnet sind. Weiters sind die Plasmabrenner 43 auch mit je einem Anschluß 54 zur Zufuhr eines Plasmagases, z.B. Helium oder Argon versehen.

Weiters ist in jedem Gehäuse 40 ein aus Kupfer hergestellter Parabolspiegel 44 angeordnet, dessen Spiegelfläche poliert ist. Dabei liegen die Achsen der Ausströmöffnungen der Kammern der Plasmabrenner 43 in der Brennachse des zugehörigen Parabolspiegels 44.

Unterhalb eines jeden Plasmabrenners 43 ist ein mit Kanälen 45 zur Führung eines Kühlmediums versehener Kupferblock 46 angeordnet, an dessen Oberseite ein Wolframtarget 46' eingepreßt ist, auf dem der Plasmastrahl auftrifft. Der Kupferblock 46 ist mit einem zweiten Anodenanschluß versehen und an die Spannungsquelle angeschlossen.

Die Gehäuse 40 sind an den dem Parabolspiegel 44 gegenüberliegenden Bereichen Öffnungen 47 angeordnet, zwischen denen die Aufnahme 41 für das zu sterilisierende Werkstück 42 angeordnet ist.

Im Bereich der Aufnahme 41 ist ein weiterer Plasmabrenner 48 angeordnet, der zur Sterilisierung von Innenräumen, insbesondere engen Kanälen vorgesehen ist, und mit einer Spannungsversorgung gemäß der Fig. 3 verbunden ist. Dieser Plasmabrenner 48 ist im wesentlichen ebenfalls gemäß den Fig. 1 und 2 ausgebildet und liefert, aufgrund der Spannungsversorgung auf der Basis einer sich ständig aufladenden und über den Lichtbogen des Plasmabrenners 48 entladenden Kondensatorbatterie 30 eine Folge von Plasmaimpulsen.

Die Aufnahme 41 ist weiters über eine Leitung 49 und ein Absperrventil 50 mit einer Vakuumpumpe 51 verbunden, sodaß Hohlräume des zu sterilisierenden Werkstückes 42 evakuiert werden können, bevor mittels des Plasmabrenners 48, Plasmaimpulse in den Hohlraum eingeschossen werden.

Da der Plasmabrenner 48 mit seiner Ausströmöffnung direkt an einer Öffnung des Hohlraumes des Werkstückes 42, z.B. ein Winkelstück mit Dentalturbine, dicht anlegbar ist, kann über diesen auch ein sauerstofffreies Plasmagas, z.B. Helium oder Argon, in den vorher evakuierten Hohlraum des Werkstückes 42 eingebracht werden, wodurch sich der nachfolgend in den Hohlraum eingeschossene Plasmaimpuls ausbreiten, bzw. diesen durchlaufen kann, wodurch auch im Hohlraum eine sichere und rasche Sterilisierung ermöglicht wird.

Die Sterilisierung der Außenflächen des Werkstückes 42 erfolgt durch die vom Plasma der beiden Plasmabrenner 43 erzeugte UV-Strahlung, die durch die Parabolspiegel 44 gebündelt wird und durch die Öffnungen 47 der Gehäuse 40 nach außen dringt und auf das Werkstück auftreffen.

Dabei kann auch ein in den Fig. 4 und 5 nicht dargestellten Drehantrieb vorgesehen sein, mit dem das Werkstück um eine horizontale Achse gedreht werden kann, um eine Sterilisierung der gesamten äußeren Flächen zu ermöglichen.

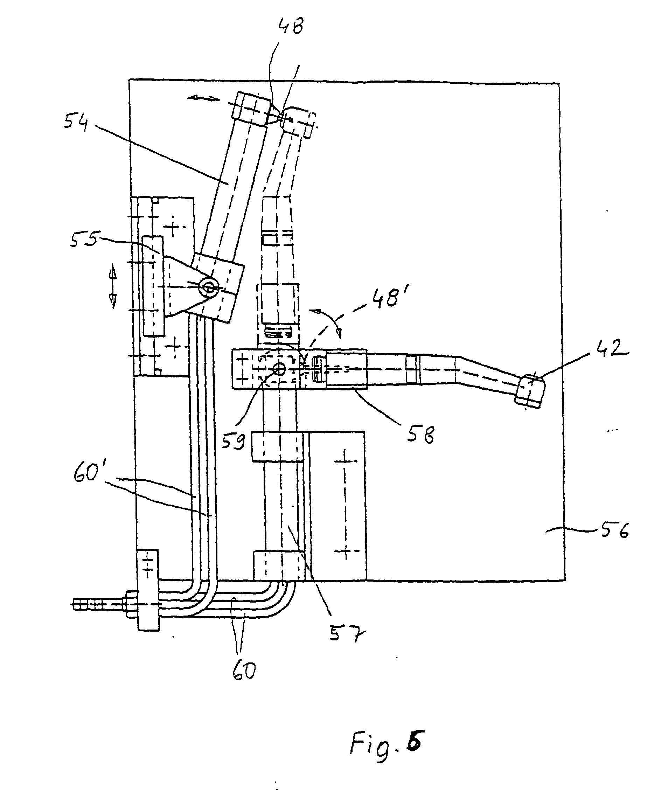

Die Fig. 6 zeigt ein weiteres Ausführungsbeispiel einer erfindungsgemäßen Einrichtung zur Sterilisierung von Werkstücken, insbesondere von Hohlräumen.

Bei dieser Ausführungsform sind zwei Plasmabrenner 48, 48' vorgesehen, von denen einer 48 an einem Schwenkarm 54 gehalten ist, der in einer 48' im wesentlichen vertikal verstellbaren Halterung 55 schwenkbar gehalten ist.

Auf dem die Halterung 55 verstellbar tragenden Gestell 56 ist weiters ein Halter 57 befestigt, an dem eine Aufnahme 58 für das zu sterilisierende Werkstück 42, z.B. ein Winkelstück mit Dentalturbine, um eine horizontale Achse 59 schwenkbar gehalten ist.

In dieser Aufnahme 58 ist auch einer der beiden Plasmabrenner 48' gehalten, sodaß sich dieser mit der Aufnahme 58 mitbewegt.

Grundsätzlich ist es aber auch möglich, den zweiten Plasmabrenner 48' in einer Lage fest zu montieren, in der die Achse seiner Ausströmöffnung koaxial mit der Achse einer Öffnung des Hohlraumes des Werkstückes 42 ausgerichtet ist, wenn sich dieses in einer bestimmten vorgegeben Lage, im dargestellten Fall in der strichliert dargestellten vertikalen Lage, befindet, in der die Sterilisation erfolgt.

Das Werkstück 42 wird in die Aufnahme 58 so befestigt, daß die Mündung der Ausströmöffnung des Plasmabrenners 48 dicht an der Öffnung des Hohlraumes des Werkstückes 42 anliegt.

Der im Schwenkarm 54 gehaltene Plasmabrenner 48 wird dicht an die eine zweite Öffnung des Hohlraumes des Werkstückes 42 angelegt.

Der Anschluß der nicht dargestellten Spannungsversorgung, deren Aufbau jenem in der Fig. 3 dargestellten entspricht, erfolgt über flexible Leitungen 60, 60' die auch für die Führung eines Kühlmediums dienen. Weiters können auch noch Anschlußleitungen zur Zufuhr eines Plasmagases zu den beiden Plasmabrennern 48, 48' vorgesehen sein.

Die beiden Plasmabrenner 48, 48' sind an zwei getrennte Spanungsversorgungen angeschlossen die miteinander synchronisiert sind, jedoch im wesentlichen in Gegenphase arbeiten.

Dabei ist vorgesehen, daß zuerst der Plasmabrenner 48' einen Plasmaimpuls in den Hohlraum des Werkstücke 42 einschießt, wodurch allfällige Flüssigkeitsreste aus dem Hohlraum aufgrund der hohen kinetischen Energie des Plasmaimpulses ausgetrieben werden, wobei der Plasmabrenner 48 vom Werkstück noch distanziert gehalten werden kann. Anschließend wird der Plasmabrenner 48 zur Anlage an dem Werkstück 42 gebracht und der Plasmabrenner 48 mit seiner zugehörigen Spannungsversorgung verbunden.

Aufgrund des Betriebs der beiden Spannungsversorgungen in Gegenphase kommt es in der Folge zu einem wechselweisen Einschießen von Plasmaimpulsen in den Hohlraum des Werkstückes 42, wodurch dieser rasch und sicher sterilisiert wird, auch wenn sich dieser über eine größere Länge erstreckt.

Wie bereits erwähnt kommt es dabei aufgrund der nur sehr kurzen Einwirkdauer der Plasmaimpulse von 10⁻⁵ bis 10⁻⁴sec. Zu keiner nennenswerten Belastung empfindlicher Teile, wie z.B. Dichtungen und Lager.

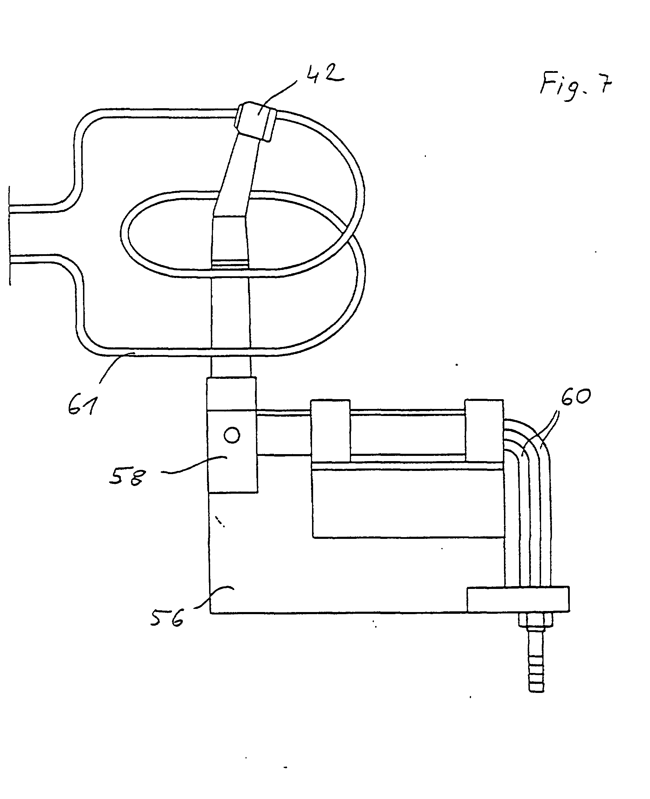

Die Fig. 7 zeigt eine Variante der Ausführungsform nach der Fig. 6 mit lediglich einem Plasmabrenner, der in der Aufnahme 58 angeordnet ist. Dabei kann die Aufnahme 58, wie bei der Ausführungsform nach der Fig. 6, schwenkbar gehalten sein, doch ist dies nicht unbedingt erforderlich. Wesentlich ist lediglich, daß die Mündung der Öffnung der Kammer des Plasmabrenners, dessen Aufbau jenem des Plasmabrenners nach den Fig. 1 und 2 entsprechen kann, dicht an den Rand der Öffnung des Hohlraumes des Werkstückes 42 anpreßbar ist.

Oberhalb der Aufnahme 58 ist eine Spule 61 angeordnet, die mittels einer nicht dargestellten HF-Signalquelle mit HF-Signalen beaufschlagbar ist.

Diese HF-Signalquelle wird in Abhängigkeit von der über die Leitungen 60 mit dem Plasmabrenner verbunden Spannungsversorgung, deren Aufbau dem in der Fig. 3 dargestellten Aufbau entspricht, angesteuert, sodaß mit dem Zünden der Anoden-Kathoden-Strecke gleichzeitig auch die Spule 61 mit HF-Signalen beaufschlagt wird.

Weiters ist der Plasmabrenner über eine nicht dargestellte Leitung mit einer Gasversorgung verbunden, die ein sauerstofffreies Plasmagas, z.B. Argon, Helium od. dgl. liefert.

Zum Sterilisieren, wird das entsprechende Werkstück, z.B. ein Winkelstück mit Dentalturbine, in der Aufnahme 58 befestigt und anschließend über den Plasmabrenner mit Plasmagas gespült. Danach wird der Plasmabrenner gezündet, wobei die Zündung im Hinblick auf die gleichzeitige Beaufschlagung der Spule 61 mit HF-Signalen zweckmäßigerweise mittels der in der Fig. 3 angedeuteten Zündeinrichtung erfolgt, wobei mit der Triggerung dieser Zündeinrichtung auch die Beaufschlagung der Spule 61 mit HF-Signalen erfolgt.

Der auf diese Weise ausgelöste und in den Hohlraum des Werkstückes 42 eingeschossene Plasmaimpuls pflanzt sich in diesem fort, bzw. durchläuft diesen, wobei das HF-Feld der Spule 61 unterstützend wirkt.

Auf diese Weise wird eine sichere und rasche Sterilisierung erreicht, wobei eine sehr weitgehende Schonung empfindlicher Teile, wie sie z.B. in einer Dentalturbine vorhanden sind, gewährleistet ist.

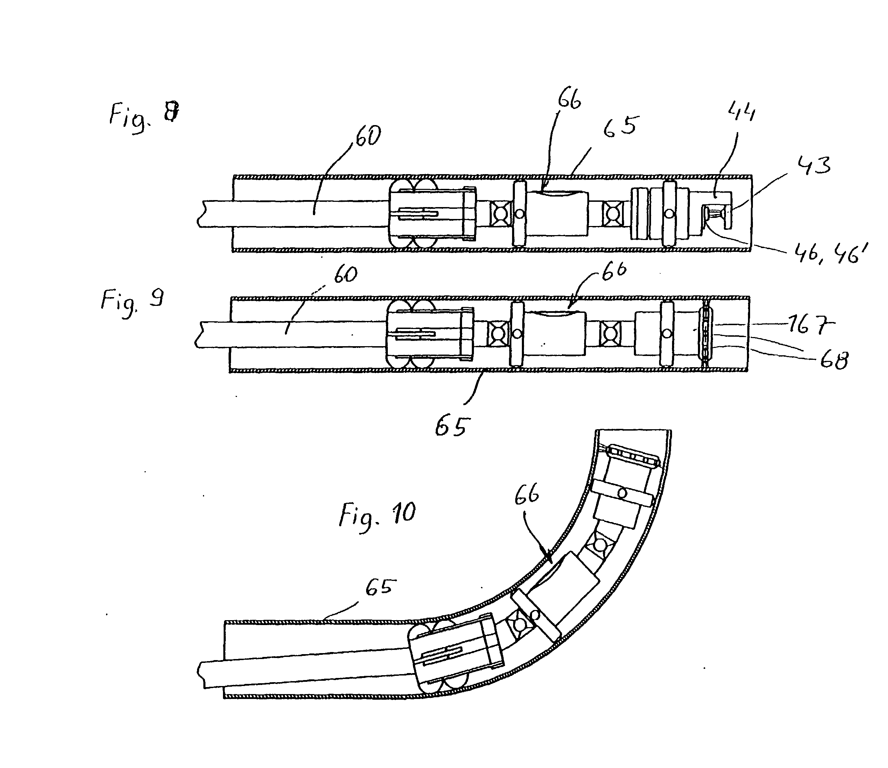

Bei der Ausführungsform nach der Fig. 8 ist ein Plasmabrenner 43 vorgesehen, dessen Ausströmöffnung koaxial mit der Brennlinie eines Parabolspiegels 44 ausgerichtet ist und mit einem mit einem Wolframtarget 46' versehen Kupferblock 46 zusammenwirkt, wobei diese Anordnung, abgesehen vom fehlenden Gehäuse, im wesentlichen der in der Fig. 4 dargestellten entspricht. Allerdings ist dabei der Parabolspiegel 44 mit einem Antrieb versehen und rotiert um den Plasmabrenner 43. Diese Anordnung ist auf einem in eine Rohrleitung 65 einsetzbaren und in dieser axial bewegbaren Molch 66 gehalten, der mittels eines eigenen Antriebes in der Rohrleitung 65 verfahrbar ist. Dabei ist auch eine Kühlung des Kupferblockes 46 und des Plasmabrenners 43 vorgesehen, wobei die entsprechenden Versorgungsleitungen 60 vom Molch 66 gezogen werden.

Die Spannungsversorgung des, zweckmäßigerweise mit einem Plasmagas, z.B. Argon, Helium, Stickstoff oder Mischungen von Gasen, beaufschlagten Plasmabrenners 43 liefert im stationären Betrieb eine im wesentlichen konstante, die Brennspannung des Lichtbogens des Plasmabrenners 43 übersteigende Spannung.

Die Sterilisierung der Innenwand der Rohrleitung 65 erfolgt dabei durch die vom Plasma erzeugte UV-Strahlung, die durch den Parabolspiegel 44 auf die Innenwand reflektiert wird.

Die Fig. 9 zeigt eine weitere Ausführungsform einer Einrichtung zum Sterilisieren von Rohrleitungen 65. Dabei ist am Kopf eines im Inneren der Rohrleitung 65 axial verfahrbaren Molches 66 ein Plasmabrenner 167 angeordnet, dessen Kammer in üblicher Weise eine Anode und eine Kathode angeordnet sind, mit einer Vielzahl von über den Umfang verteilt angeordneten Ausströmöffnungen 68 versehen ist.

Bei dieser Einrichtung ist der Plasmabrenner 167 mit einer der Fig. 3 entsprechenden Spannungsversorgung verbunden, sodaß dieser eine Folge von Plasmaimpulsen liefert, die aufgrund der extrem raschen Erwärmung des Plasmas beim Zünden des Lichtbogens, mit sehr hoher Geschwindigkeit aus den Ausströmöffnungen 68 austreten und auf die Innenwand der Rohrleitung auftreffen und diese sterilisieren. Dabei werden mikroskopische Partikel, wie es Bakterien und Viren einem hohen thermischen Impuls und der UV-Strahlung, sowie auch der kinetischen Energie der Plasmaimpulse ausgesetzt und so eine sichere Sterilisierung erreicht.

Da die Einwirkdauer der Plasmaimpulse nur sehr kurz ist und z.B. nur 10⁻⁵ bis 10⁻⁴sec beträgt, kommt es zu keiner nennenswerten thermischen Belastung der Rohrleitung.

Wie aus Fig. 10 zu ersehen ist, kann der den Plasmabrenner 167 tragende Molch 66 auch Krümmungen der Rohrleitung 65 durchfahren.

Der Plasmabrenner 167 ist ähnlich dem Plasmabrenner, bzw. Modul gemäß der österreichischen Patentanmeldung A 135/98 ausgebildet. Dabei begrenzt eine Sackbohrung einer Spannhülse 64' eine Kammer 27', von der aus radiale Bohrungen 16' nach außen führen.

Weiters ist in der Spannhülse 64' eine Anode 15' gehalten in deren zentrischer Bohrung eine Zentrierhülse 74' eingesetzt ist, die mit nach innen gerichteten Rippen die Kathode 19' führt. Dabei ist die Zentrierhülse 74, die aus einem Isoliermaterial, z.B. Keramik hergestellt ist, mittels einer ebenfalls aus einem elektrisch isolierenden Werkstoff hergestellte Hülse 73' und einer Schulter der Bohrung der Anode 15' in ihrer axialen Lage gehalten.

Die Kammer 27' steht über einen zwischen der Innenwand der Hülse 73' und der Kathode 19 verbleibenden Ringspalt 100 mit einer nicht dargestellten, mit einem Anschluß für eine Gaszuführleitung versehenen Kammer in Verbindung, sodaß im Betrieb sauerstofffreies Gas, z.B. Argon, Helium, Stickstoff od. dgl. in die Kammer 27' eingeleitet werden kann, wobei im Betrieb aufeinanderfolgend Spannungsimpulse an die Anode 15' und Kathode 19' gelegt werden, die die Überschlagsspannung der Anoden-Kathoden-Strecke überschreiten und zur Zündung eines Lichtbogens führen. Als Spannungsversorgung ist zweckmäßigerweise eine der Fig. 3 entsprechende vorgesehen.

## Patentansprüche

1. Verfahren zum Sterilisieren von Gegenständen bei welchem diese zumindest der Strahlung eines Plasmas ausgesetzt werden, **dadurch gekennzeichnet, dass** die zu sterilisierenden Gegenstände mit einer Folge von mittels eines intermittierenden Lichtbogens erzeugten Plasmaimpulsen beaufschlagt werden, deren Impulsdauer im Bereich zwischen 10⁻⁵ und 10⁻³ Sekunden, vorzugsweise zwischen 10⁻⁴ und 10⁻⁵ Sekunden beträgt, wobei der Lichtbogen durch Entladung einer Kondensatorbatterie mit einer Spannung die zumindest der Überschlagsspannung der Anoden-Kathoden-Strecke entspricht, erzeugt wird.

2. Verfahren nach Anspruch 1, zur Sterilisierung von enge Hohlräume aufweisenden Gegenständen, wie z.B. Dentalturbinen od. dgl., **dadurch gekennzeichnet, dass** die Hohlräume mit einem zum Erzeugen des Plasmas vorgesehenen, im wesentlichen sauerstofffreiem Gas, z.B. Helium, Argon, u.dgl., gefüllt und anschließend die Plasmaimpulse in den Hohlraum eingeschossen werden, wobei vor jedem Plasmaimpuls der Hohlraum, vorzugsweise mit Vakuumunterstützung, mit dem Gas gefüllt wird.

3. Verfahren nach Anspruch 2, **dadurch gekennzeichnet, dass** der zu sterilisierende Gegenstand während des Einschießens der Plasmaimpulse in den Hohlraum gleichzeitig einem HF-Feld ausgesetzt wird.

4. Einrichtung zur Durchführung des Verfahrens nach einem der Ansprüche 1 bis 3, bei der eine Aufnahme (41,58) für einen zu sterilisierenden Gegenstand und ein Plasmabrenner (48,48') vorgesehen sind, der eine mit einer Ausströmöffnung (16) und mit einer Gaszufuhrleitung versehene Kammer aufweist, in dereine Anode und eine Kathode, die beide an eine Spannungsversorgung angeschlossen sind, angeordnet sind, **dadurch gekennzeichnet, dass** die Spannungsversorgung durch eine Kondensatorbatterie (30) gebildet ist, die mit einer eine über der Überschlagsspannung der Anoden-Kathoden-Strecke (15, 19) liegenden Spannung liefernden Ladeschaltung (31, 32) verbunden ist und ausgangsseitig mit der Anode (15) und der Kathode (19) des Plasmabrenners (11) verbunden ist.

5. Einrichtung nach Anspruch 4**, dadurch gekennzeichnet, dass** zusätzlich zu dem mittels der Kondensatorbatterie (30) gespeisten Plasmabrenner ein weiterer Plasmabrenner (43) vorgesehen ist und sich die Achsen der Ausströmöffnungen (16) der Kammern (27) der Plasmabrenner (43) im wesentlichen in der Brennlinie eines Parabolspiegels (44) aus poliertem Kupfer befinden, der einer Öffnung (47) der die Plasmabrenner (43) samt Spiegel (44) aufnehmenden Gehäuse (40) gegenüberliegend angeordnet ist, wobei die Aufnahme (41) für den zu sterilisierenden Gegenstand (42) vor dieser Öffnung (47) angeordnet ist.

6. Einrichtung nach einem der Ansprüche 4 oder 5, **dadurch gekennzeichnet, dass** die Aufnahme (58) für den zu sterilisierenden Gegenstand (42) im Bereich einer Spule (61) angeordnet ist, die mit einer HF-Signalquelle verbindbar ist.

7. Einrichtung nach einem der Ansprüche 4 bis 6, wobei der zu sterilisierende Gegenstand einen an zwei Stellen offenen Hohlraum aufweist, **dadurch gekennzeichnet, dass** der Plasmabrenner (48,48') im Bereich der Aufnahme (58) angeordnet ist und mit dem Rand seiner Ausströmöffnung (16) an einen eine Öffnung des Hohlraumes umgebenden Rand des zu sterilisierenden Gegenstandes (42) zur Anlage bringbar ist.

8. Einrichtung nach Anspruch 7, **dadurch gekennzeichnet, dass** an die Anode (15) und die Kathode (19) zusätzlich ein separates, vorzugsweise HF-Signale lieferndes, Zündgerät (35) angeschlossen ist.

9. Einrichtung nach Anspruch, **dadurch gekennzeichnet, dass** ein zweiter Plasmabrenner (48) vorgesehen ist, der an einem Arm (54) gehalten und an die zweite Öffnung des Hohlraumes des zu sterilisierenden Gegenstandes (42) zur Anlage bringbar ist, und die Aufnahme (58) für den zu sterilisierenden Gegenstand (42) mit einer Unterdruckquelle verbindbar ist und zumindest der im Arm (54) gehaltene Plasmabrenner (48) mit einer ein sauerstofffreies Gas liefernden Gasquelle verbindbar ist.

10. Einrichtung zum Sterilisieren von Innenwänden von Rohrleitungen mittels eines Plasmabrenners (43) gemäß Anspruch 4, **dadurch gekennzeichnet, dass** ein Parabolspiegel (44) vorgesehen ist, der um eine koaxial zur Achse der Ausströmöffnung (16) des Plasmabrenners (43) verlaufende Achse drehbar gehalten und mit einem Antrieb versehen ist, wobei der Parabolspiegel (44) samt Plasmabrenner (43) in einem im Inneren des zu sterilisierenden Rohres (65) verfahrbaren Gestell, bzw. Molch (66) gehalten sind.

11. Einrichtung zum Sterilisieren von Innenwänden von Rohrleitungen mittels eines Plasmabrenners (43) gemäß Anspruch 4, **dadurch gekennzeichnet, dass** die die Anode und die Kathode aufnehmende Kammer des Plasmabrenners (167) eine Vielzahl von radialen Ausströmöffnungen (68) aufweist, wobei der Plasmabrenner (167) in einem im Inneren des zu sterilisierenden Rohres (65) verfahrbaren Gestell, bzw. Molch (66) gehalten sind.

## Claims

1. A method for sterilizing objects with which the same are subjected at least to the radiation of a plasma, **characterized in that** the objects to be sterilized are subjected to a sequence of plasma pulses produced by means of an intermitting arc, with the duration of the pulses being in the region of between 10⁻⁵ and 10⁻³ seconds, preferably between 10⁻⁴ and 10⁻⁵ seconds, with the arc being produced by the discharge of a capacitor battery with a voltage corresponding at least to the arc-over voltage of the anode-to-cathode distance.

2. A method as claimed in claim 1, for the sterilization of objects comprising narrow cavities such as dental turbines or the like, **characterized in that** the cavities are filled with a substantially oxygen-free gas such as helium, argon and the like for producing the plasma and thereafter the plasma pulses are injected into the cavity, with the cavity being filled with the gas, preferably with vacuum support, prior to each plasma pulse.

3. A method as claimed in claim 2, **characterized in that** the object to be sterilized is simultaneously subjected to an RF field during the injection of the plasma pulses into the cavity.

4. A device for performing the method according to one of the claims 1 to 3, in which a receiver (41, 58) for an object to be sterilized and a plasma torch (48, 48') are provided, which torch comprises a chamber having an outflow opening (16) and a gas supply line, in which chamber there are disposed an anode and a cathode which are both connected to a power supply, **characterized in that** the power supply is formed by a capacitor battery (30) which is connected to a charging circuit (31, 32) supplying a voltage over the arc-over voltage of the anode-to-cathode distance (15, 19) and is connected on the output side to the anode (15) and the cathode (19) of the plasma torch (11).

5. A device as claimed in claim 4, **characterized in that** in addition to the plasma torch supplied by means of the capacitor battery (30) there is a further plasma torch (43) and the axes of the outflow openings (16) of the chambers (27) of the plasma torches (43) are situated substantially in the focal line of a parabolic reflector (44) made of polished copper which is arranged opposite of an opening (47) of a housing (40) receiving the plasma torch (43) plus reflector (44), with the receiver (41) for the object (42) to be sterilized being situated in front of said opening (47).

6. A device as claimed in one of the claims 4 or 5, **characterized in that** the receiver (58) for the object (42) to be sterilized is arranged in the region of a coil (61) which is connectable with an RF signal source.

7. A device as claimed in one of the claims 4 to 6, with the object to be sterilized comprising a cavity open at two places, **characterized in that** the plasma torch (48, 48') is arranged in the region of the receiver (58) and can be brought with the edge of its outflow opening (16) into contact with an edge of the object (42) to be sterilized, which edge encloses an opening of the cavity.

8. A device as claimed in claim 7, **characterized in that** a separate ignition device (35) which preferably supplies RF signals is additionally connected to the anode (15) and the cathode (19).

9. A device as claimed in claim 7, **characterized in that** a second plasma torch (48) is provided which is held on an arm (54) and can be brought into contact with the second opening of the cavity of the object (42) to be sterilized, and the receiver (58) for the object (42) to be sterilized can be connected with a low-pressure source and at least the plasma torch (54) held in the arm can be connected to a gas source supplying an oxygen-free gas.

10. A device for sterilizing interior walls of pipelines by means of a plasma torch (43) according to claim 4, **characterized in that** a parabolic reflector (44) is provided which is held in a rotatable manner about an axis extending coaxially to the axis of the outflow opening (16) of the plasma torch (43) and is provided with a drive, with the parabolic reflector (44)plus plasma torch (43) being held in a frame or go-devil (66) movable within the pipe (65) to be sterilized.

11. A device for sterilizing interior walls of pipelines by means of a plasma torch (43) according to claim 4, **characterized in that** the chamber of the plasma torch (167) receiving the anode and cathode comprises a plurality of radial outflow openings (68), with the plasma torch (167) being held in a frame or go-devil (66) movable within the pipe (65) to be sterilized.

## Revendications

1. Procédé pour la stérilisation d'objets, dans lequel ceux-ci sont soumis au moins au rayonnement d'un plasma, **caractérisé en ce que** les objets à stériliser sont exposés à une séquence d'impulsions de plasma produites au moyen d'un arc électrique intermittent, dont la durée d'impulsions se situe entre 10⁻⁵ et 10⁻³ secondes, de préférence entre 10⁻⁴ et 10⁻⁵ secondes, l'arc électrique étant produit par la décharge d'une batterie de condensateurs à une tension correspondant au moins à la tension d'éclatement de la distance anode-cathode.

2. Procédé selon la revendication 1 pour la stérilisation d'objets présentant des cavités étroites, par exemple de turbines dentaires ou similaires, **caractérisé en ce que** les cavités sont remplies d'un gaz destiné à produire le plasma et ne contenant sensiblement pas d'oxygène, par exemple de l'hélium, de l'argon ou similaire, et les impulsions de plasma sont ensuite projetées dans la cavité, laquelle cavité est remplie du gaz avant chaque impulsion de plasma, de préférence avec l'assistance d'une aspiration.

3. Procédé selon la revendication 2, **caractérisé en ce que** l'objet à stériliser est exposé à un champ à haute fréquence en même temps que les impulsions de plasma sont projetées dans la cavité.

4. Dispositif pour la mise en oeuvre du procédé selon l'une quelconque des revendications 1 à 3, dans lequel il est prévu un logement (41, 58) pour l'objet à stériliser et une torche à plasma (48, 48'), qui présente une chambre pourvue d'une ouverture de sortie (16) et d'une conduite d'alimentation en gaz, dans laquelle sont disposées une anode et une cathode raccordées toutes deux à une alimentation électrique, **caractérisé en ce que** l'alimentation électrique est formée par une batterie de condensateurs (30) qui est connectée à un circuit de charge (31, 32) fournissant la tension appliquée par l'intermédiaire de la tension d'éclatement sur la distance anode-cathode (15, 19) et qui est reliée du côté de la sortie avec l'anode (15) et la cathode (19) de la torche à plasma (11).

5. Dispositif selon la revendication 4, **caractérisé en ce qu'**outre la chambre à plasma alimentée au moyen de la batterie de condensateurs (30), il est prévu une autre torche à plasma (43) et les axes des ouvertures de sortie (16) des chambres (27) des torches à plasma (43) se trouvent sensiblement dans l'axe focal d'un miroir parabolique (44) en cuivre poli, qui est disposé face à une ouverture (47) des boîtiers (40) contenant les torches à plasma (43) et les miroirs (44), le logement (41) de l'objet à stériliser (42) étant disposé devant cette ouverture (47).

6. Dispositif selon l'une quelconque des revendications 4 ou 5, **caractérisé en ce que** le logement (58) pour l'objet à stériliser (42) est disposé à proximité d'une bobine (61) qui peut être connectée à une source de signal à haute fréquence.

7. Dispositif selon l'une quelconque des revendications 4 à 6, dans lequel l'objet à stériliser présente une cavité ouverte à deux endroits, **caractérisé en ce que** la torche à plasma (48, 48') est disposée au niveau du logement (58) et peut être mise en contact avec le bord de son ouverture de sortie (16) sur un bord de l'objet à stériliser (42) entourant l'ouverture de la cavité.

8. Dispositif selon la revendication 7, **caractérisé en ce qu'**il est prévu au niveau de l'anode (15) et de la cathode (19) un appareil d'allumage (35) délivrant de préférence des signaux à haute fréquence.

9. Dispositif selon la revendication 7, **caractérisé en ce qu'**il est prévu une deuxième torche à plasma (48) qui est retenue sur un bras (54) et peut être mise en contact avec la deuxième ouverture de la cavité de l'objet à stériliser (42), et le logement (58) pour l'objet à stériliser (42) peut être mis en communication avec une source de vide et au moins la torche à plasma (48) retenue par le bras (54) peut être mise en communication avec une source de gaz fournissant un gaz sans oxygène.

10. Dispositif pour la stérilisation des parois intérieures de tuyaux au moyen d'une torche à plasma (43) selon la revendication 4, **caractérisé en ce qu'**il est prévu un miroir parabolique (44) qui est retenu avec possibilité de rotation autour d'un axe coaxial de l'axe de l'ouverture de sortie (16) de la torche à plasma (43) et pourvu d'un entraînement, lequel miroir parabolique (44) est retenu avec la torche à plasma (43) dans un bâti ou écouvillon (66) pouvant être déplacé à l'intérieur du tuyau à stériliser (65).

11. Dispositif pour la stérilisation des parois intérieures de tuyaux au moyen d'une torche à plasma (43) selon la revendication 4, **caractérisé en ce que** la chambre de la torche à plasma (167) recevant l'anode et la cathode présente une pluralité d'ouvertures de sortie radiales (68), la torche à plasma (167) étant retenue dans un bâti ou écouvillon (66) pouvant être déplacé à l'intérieur du tuyau à stériliser (65).
